# EUROPÄISCHE PATENTSCHRIFT

(11) **EP 0 477 531 B1**
(45) Veröffentlichungstag und Bekanntmachung des Hinweises auf die Patenterteilung: **30.11.1994**
(21) Anmeldenummer: 91113892.3
(22) Anmeldetag: 20.08.1991
(51) Int. Cl.: C07C 43/04, C07C 41/46

(54) **Verfahren zur Inhibierung und Zerstörung von Peroxiden in Dialkylethern**
Process for inhibiting and destroying peroxides in dialkyl ethers
Procédé pour l'inhibition et pour la dégradation de peroxides dans les ethers dialkyliques

(30) Priorität: 21.09.1990 DE 4029875
(43) Veröffentlichungstag der Anmeldung: 01.04.1992
(73) Patentinhaber: HOECHST AKTIENGESELLSCHAFT, 65926 Frankfurt am Main (DE)
(72) Erfinder: Gradl, Reinhard, Dr., W-5042 Erftstadt (DE); Erpenbach, Heinz, Dr., W-5000 Köln (DE); Weferling, Norbert, Dr., W-5030 Hürth (DE); Jägers, Erhard, Dr., W-5303 Bornheim (DE); Seidel, Andreas, Dr., W-5000 Köln (DE)

(56) Entgegenhaltungen:
- DE-A- 3 046 148
- US-A- 2 279 277
- US-A- 2 912 366
- US-A- 3 003 002
- US-A- 3 483 260

## Beschreibung

Die Erfindung betrifft ein Verfahren zur Inhibierung und Zerstörung von Peroxiden in Dialkylethern mit der allgemeinen Formel R₁OR₂, worin R₁ und R₂ Alkylgruppen mit 1 bis 6, insbesondere 2 oder 3 C-Atomen bedeuten.

Ether werden in der Technik als Extraktionsmittel oder als Reaktant bei Carbonylierungsreaktionen eingesetzt.

Während des Transportes und der Zwischenlagerung werden in Dialkylethern Peroxide gebildet, die sich explosionsartig zersetzen können. Zur Inhibierung und Zerstörung von Peroxiden werden den Dialkylethern Radikalfänger oder Reduktionsmittel wie beispielsweise 2,6-Di-tert-butyl-4-methylphenol, Natriumsulfit, Eisen(II)sulfat, Kaliumpyrosulfit, Triethylentetramin oder Natriumthiocarbamat zugegeben. Nachteilig hierbei ist, daß diese Verbindungen als Verunreinigung in die Nachfolgeverfahren eingeschleppt werden oder vor ihrem Gebrauch durch eine Destillation oder Wäsche abgetrennt werden müssen.

Peroxide in Dialkylethern können mit dem Perex-Test®, veröffentlicht von der Firma E. Merck, Postfach 41 19, 6100 Darmstadt 1, Bundesrepublik Deutschland, in einfacher Weise bestimmt werden.

Bei der Carbonylierung von Methanol, Methylacetat oder Dimethylether fallen polymere, teerige Nebenprodukte an, welche die Wirksamkeit der Edelmetallkatalysatoren herabsetzen und deshalb aus dem Verfahren ohne Edelmetallverluste ausgeschleust werden müssen. In der DE-32 20 226 C2 (US-A-4,476,238) wird eine Extraktionstrennung des Edelmetallkatalysators von dem polymeren, teerigen Nebenprodukt mittels Dialkylethern beschrieben.

Es war die Aufgabe gestellt, ein Verfahren zur Inhibierung und Zerstörung von Peroxiden in Dialkylethern mit der allgemeinen Formel R₁OR₂, worin R₁ und R₂ Alkylgruppen mit 1 bis 6 C-Atomen bedeuten, anzugeben, durch das keine Einschleppung von artfremden Verbindungen erfolgt.

Überraschend wurde nun gefunden, daß man eine wirksame Inhibierung und Zerstörung von Peroxiden in Dialkylethern erzielt, wenn man den Dialkylethern als Inhibitor quartäres Alkyliodid eines Elementes der fünften Hauptgruppe des Periodischen Systems der Elemente zusetzt.

Das Verfahren der Erfindung kann weiterhin bevorzugt und wahlweise dadurch gekennzeichnet sein, daß man
a) aus den Elementen der fünften Hauptgruppe des Periodischen Systems der Elemente Phosphor, Stickstoff oder Arsen auswählt;
b) ein quartäres Alkyliodid der allgemeinen Formel

   [CH₃Alk₃P]I,

   worin Alk Alkylgruppen mit 1 bis 16, vorzugsweise mit 4 bis 8 C-Atomen bedeuten, zusetzt;
c) dem Dialkylether als Inhibitor 25 bis 250, insbesondere 50 bis 100 mg quartäres Alkyliodid/1000 g Dialkylether zusetzt;
d) den Inhibitor als Lösung in Alkohol, insbesondere in Methanol, Ethanol oder i-Propanol zusetzt;
e) den Inhibitor als 0,5 bis 10, insbesondere als 1 bis 5 gew.-%ige alkoholische Lösung zusetzt.

Ein weiterer Vorteil der erfindungsgemäßen Inhibierung und Zerstörung von Peroxiden in Dialkylethern liegt in der optischen Überwachung der Dialkylether. Die durch Peroxid gebildete Iodmenge führt zu einer Gelbfärbung, deren Intensität ein Indikator für die Peroxidmenge ist.

Die nachfolgenden Beispiele erläutern unsere Erfindung näher.

### Beispiel 1

Je 200 g peroxidfreier Diisopropylether wurden mit 25 ppm des angegebenen Methyltrialkylphosphoniumiodids (als 1 gew.-%ige Lösung in Methanol) versetzt und in einem farblosen Glaskolben unter Sonneneinstrahlung einem gelinden Luftstrom ausgesetzt. Der Peroxid-Gehalt wurde mit dem PEREX-Test bestimmt.

In der Tabelle 1 sind die Ergebnisse zusammengefaßt.

**Tabelle 1**

| Inhibitor [25 mg] | Peroxid-Gehalt [ppm] Begasungszeit in Tagen | | | |
|---|---|---|---|---|
| | 0 | 4 | 5 | 6 |
| (CH₃)₄PI | 0 | 15 | 25 | 50 |
| CH₃(n-C₄H₉)₃PI | 0 | 0 | 0 | 50 |
| CH₃(n-C₈H₁₇)₃PI | 0 | 0 | 0 | 50 |
| ohne Inhibitor | 0 | 25 | 50 | 60 |

### Beispiel 2

Das Beispiel 1 wurde mit folgenden Änderungen wiederholt:
150 g peroxidfreier Diethylether wurden mit 50 ppm CH₃(n-C₄H₉)₃PI (als 3,5 gew.-%ige Lösung in Methanol) versetzt.

**Tabelle 2**

| Inhibitormenge [ppm] | Peroxid-Gehalt [ppm] Begasungszeit in Tagen | | | | | |
|---|---|---|---|---|---|---|
| | 0 | 6 | 12 | 19 | 25 | 37 |
| 50 | 0 | 0 | 0 | 0 | 0 | 0 |
| 0 | 0 | 2 | 50 | 100 | 250 | 500 |

### Beispiel 3

Das Beispiel 1 wurde mit folgenden Änderungen wiederholt:
Je 150 g peroxidfreier Diisopropylether wurden mit wechselnder Menge an CH₃(n-C₄H₉)₃PI (als 1 gew.-%ige Lösung in Methanol) versetzt.

**Tabelle 3**

| Inhibitormenge [ppm] | Peroxid-Gehalt [ppm] Begasungszeit in Tagen | | | | | |
|---|---|---|---|---|---|---|
| | 0 | 6 | 12 | 19 | 25 | 37 |
| 50 | 0 | 0 | 0 | 0 | 0 | 0 |
| 75 | 0 | 0 | 0 | 0 | 0 | 0 |

## Patentansprüche

1. Verfahren zur Inhibierung und Zerstörung von Peroxiden in Dialkylethern mit der allgemeinen Formel R₁OR₂, worin R₁ und R₂ Alkylgruppen mit 1 bis 6 C-Atomen bedeuten, dadurch gekennzeichnet, daß man den Dialkylethern als Inhibitor quartäres Alkyliodid eines Elementes der fünften Hauptgruppe des Periodischen Systems der Elemente zusetzt.

2. Verfahren nach Anspruch 1, dadurch gekennzeichnet, daß man aus den Elementen der fünften Hauptgruppe des Periodischen Systems der Elemente Phosphor, Stickstoff oder Arsen auswählt.

3. Verfahren nach Anspruch 1 oder 2, dadurch gekennzeichnet, daß man ein quartäres Alkyliodid der allgemeinen Formel
[CH₃Alk₃P]I,
worin Alk Alkylgruppen mit 1 bis 16, vorzugsweise mit 4 bis 8 C-Atomen bedeuten, zusetzt.

4. Verfahren nach einem der Ansprüche 1 bis 3, dadurch gekennzeichnet, daß man dem Dialkylether als Inhibitor 25 bis 250, insbesondere 50 bis 100 mg quartäres Alkyliodid/1000 g Dialkylether zusetzt.

5. Verfahren nach einem der Ansprüche 1 bis 4, dadurch gekennzeichnet, daß man den Inhibitor als Lösung in Alkohol, insbesondere in Methanol, Ethanol oder i-Propanol zusetzt.

6. Verfahren nach Anspruch 5, dadurch gekennzeichnet, daß man den Inhibitor als 0,5 bis 10, insbesondere als 1 bis 5 gew.-%ige alkoholische Lösung zusetzt.

## Claims

1. A process for inhibiting and destroying peroxides in dialkyl ethers having the general formula R₁OR₂ in which R₁ and R₂ are alkyl groups having 1 to 6 carbon atoms, which comprises adding, as inhibitor, a quaternary alkyl iodide of an element of the fifth main group of the Periodic Table of the Elements to the dialkyl ethers.

2. The process as claimed in claim 1, wherein phosphorus, nitrogen or arsenic is selected from the elements of the fifth main group of the Periodic Table of the Elements.

3. The process as claimed in claim 1 or 2, wherein a quaternary alkyl iodide of the general formula
[CH₃alk₃P]I,
in which alk is an alkyl group having 1 to 16, preferably 4 to 8, carbon atoms, is added.

4. The process as claimed in one of claims 1 to 3, wherein from 25 to 250, in particular 50 to 100 mg of quaternary alkyl iodide/1000 g of dialkyl ether are added as inhibitor to the dialkyl ether.

5. The process as claimed in one of claims 1 to 4, wherein the inhibitor is added as a solution in alcohol, in particular in methanol, ethanol and isopropanol.

6. The process as claimed in claim 5, wherein the inhibitor is added as a from 0.5 to 10, in particular 1 to 5 % strength by weight alcoholic solution.

## Revendications

1. Procédé d'inhibition et de destruction de peroxydes dans des éthers dialkyliques de formule générale R₁OR₂, dans laquelle R₁ et R₂ représentent des groupements alkyle en C₁-C₆, caractérisé en ce qu'on ajoute à l'éther dialkylique un iodure d'alkyle quaternaire d'un élément du cinquième groupe de la classification périodique des éléments, en tant qu'inhibiteur.

2. Procédé selon la revendication 1, caractérisé en ce qu'on choisit parmi les éléments du cinquième groupe de la classification périodique des éléments le phosphore, l'azote ou l'arsenic.

3. Procédé selon la revendication 1 ou 2, caractérisé en ce qu'on ajoute un iodure d'alkyle quaternaire de formule générale :
[CH₃Alk₃P]I,
dans laquelle Alk représente un groupement alkyle en C₁-C₁₆, de préférence C₄-C₈.

4. Procédé selon l'une quelconque des revendications 1 à 3, caractérisé en ce qu'on ajoute à l'éther dialkylique en tant qu'inhibiteur 25 à 250 mg, de préférence 50 à 100 mg d'iodure d'alkyle quaternaire pour 1 000g d'éther dialkylique.

5. Procédé selon l'une des revendications 1 à 4, caractérisé en ce qu'on ajoute l'inhibiteur sous forme d'une solution dans un alcool, de préférence le méthanol, l'éthanol ou l'isopropanol.

6. Procédé selon la revendication 5, caractérisé en ce qu'on ajoute l'inhibiteur sous forme d'une solution alcoolique à 0,5 à 10 % en masse, de préférence 1 à 5 % en masse.
